# EUROPEAN PATENT APPLICATION

(11) **EP 4 361 251 A1**
(43) Date of publication of application: **01.05.2024**
(21) Application number: 23204699.5
(22) Date of filing: 19.10.2023
(51) Int. Cl.: C12N 1/14, C12N 9/90, C12N 15/80, C12P 35/00

(54) **DEACETOXYCEPHALOSPORIN C-PRODUCING MICROORGANISM FOR 7-ADCA PRODUCTION WITH HIGH CONCENTRATION AND HIGH PURITY AND PRODUCTION METHOD USING THE SAME**

(30) Priority: 26.10.2022 KR 20220139497
(71) Applicant: Amicogen, Inc., Jinju-si, Gyeongsangnam-do 52840 (KR)
(72) Inventor: Piao, Zhe, 52840 Jinjusi (KR); Yun, Young Sung, 52840 Jinjusi (KR); Lee, Hyeon Seo, 52840 Jinjusi (KR); Choi, Yeon Hee, 52840 Jinjusi (KR); Kang, Mi Suk, 52840 Jinjusi (KR); Piao, Xue Mei, 52840 Jinjusi (KR); Kim, You Mi, 52840 Jinjusi (KR); Lee, Ji Su, 52840 Jinjusi (KR); Li, Hong Xian, 52840 Jinjusi (KR); Seo, Dong Il, 52840 Jinjusi (KR); Jeong, Dong Won, 52840 Jinjusi (KR); Kim, Seung Ki, 52840 Jinjusi (KR)
(74) Representative: Arnold & Siedsma

(57) **Abstract**

Disclosed herein are a microorganism with excellent deacetoxycephalosporin C (DAOC) productivity and a use thereof. A mutant microorganism with improved DAOC productivity and a use thereof for producing 7-aminodeacetoxycephalosporanic acid (7-ADCA) are provided.

## Description

### BACKGROUND OF THE INVENTION

### 1. Field of the Invention

The present application relates to a microorganism with excellent productivity of deacetoxycephalosporin C (DAOC) and a use thereof and, more specifically, to a mutant microorganism with improved productivity of DAOC and a use thereof for producing 7-aminodeacetoxycephalosporanic acid (7-ADCA).

### 2. Description of the Related Art

Cephalosporin C (hereinafter, referred to as "CPC"), which is a family of beta-lactam antibiotics, is produced by microorganisms such as *Acremonium chrysogenum.* CPC exhibits antibiotic activity against Gram-negative bacteria by inhibiting cell wall synthesis, but due to its very weak activity, finds the main application in preparing raw materials for semi-synthetic cephalosporin antibiotics (hereinafter referred to as "cephalosporins").

Raw materials of cephalosporins can be mainly divided into 7-aminocephalosporanic acid (hereinafter abbreviated to "7-ACA"), deacetyl 7-aminocephalosporanic acid (hereinafter abbreviated to "D-7-ACA"), and 7-aminodeacetoxycephalosporanic acid (hereinafter abbreviated to "7-ADCA").

Currently used for industrial production of 7-ADCA is a chemical conversion method to expand penam ring structures found in penicillin-type molecules to cephem rings. However, the chemical conversion method for producing 7-ADCA by expansion to cephem rings employs organic solvents such as toluene, which leads to the generation of a lot of toxic wastes, suffering from the disadvantage of producing environmental contamination and increasing the cost for treatment of wastewater.

Conventional recombinant strains for producing DAOC at high concentrations are disadvantageous in that the product purity decreases due to the byproduct deacetylcephalosporin C (DAC) generated during DAOC production.

There is therefore a need for the development of a strain that can produce the target substance DAOC at high purity with productivity increased for DAOC and decreased for byproducts.

### [Related Art Document]

### Patent literature

Korean Patent No. 10-2194740

### SUMMARY OF THE INVENTION

Provided in the present application are a microorganism with excellent productivity of deacetoxycephalosporin C (DAOC) and a use thereof.

An aspect provides a novel DAOC-producing microorganism. In an embodiment, the DAOC-producing microorganism may be a mutant *Acremonium chrysogenum* microorganism having *Streptomyces clavuligerus-*derived aminotransferase introduced thereinto. The mutant *Acremonium chrysogenum* microorganism may have DAOC productivity.

Another aspect provides a method for constructing an *Acremonium chrysogenum* microorganism with improved DAOC productivity and/or a method for improving DAOC productivity in an *Acremonium chrysogenum* microorganism, each method comprising a step of introducing a *Streptomyces clavuligerus-derived* aminotransferase gene into the *Acremonium chrysogenum* microorganism.

A further aspect provides a method for producing DAOC, the method comprising a step of culturing the mutant *Acremonium chrysogenum* microorganism.

### DETAILED DESCRIPTION

This application provides a mutant microorganism having improved productivity of deacetoxycephalosporin C (DAOC) and a use thereof. With high productivity of DAOC and low yield of byproducts (e.g., deacetylcephalosporin C (DAC), etc.), the mutant microorganism is characterized by the production of DAOC at high purity.

### Definition of Terms

As used herein, the phrase that a polynucleotide (interchangeably used with "gene" or "nucleic acid molecule") or a polypeptide (interchangeably used with "protein" or "enzyme") comprises, consists (essentially) of, or represented by "a certain nucleic acid sequence or a certain amino acid sequence" may mean that the polynucleotide or a polypeptide essentially contains the certain nucleic acid sequence or amino acid sequence, or may be construed to comprise a "substantially identical sequence" resulting from addition of a mutation (deletion, substitution, alteration, and/or addition) to the certain nucleic acid or amino acid sequence to the extent of retaining the original function and/or desired function of the polynucleotide or polypeptide.

In an embodiment, a polynucleotide or a polypeptide" comprises, consists (essentially) of, or represented by "a certain nucleic acid sequence or a certain amino acid sequence" may mean that the polynucleotide or the polypeptide
(i) essentially contains the certain nucleic acid sequence or amino acid sequence, or
(ii) essentially consists of or comprises a nucleic acid sequence or an amino acid sequence having 60% or higher, 70% or higher, 80% or higher, 85% or higher, 90% or higher, 91% or higher, 92% or higher, 93% or higher, 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher, or 99.9% or higher (exclusive of 100% in some cases) sequence identity with the certain nucleic acid sequence or amino acid sequence and retains the original function and/or a desired function of the certain sequence. In an embodiment, the desired function may include conferring DAOC productivity to a host cell or increasing DAOC productivity of a host cell and/or reducing a production of a byproduct during DAOC production.

As used herein, the term "sequence identity" may refer to a degree of match between two nucleic acid sequences or between two amino acid sequences and may be expressed as a percentage (%). As for identity with nucleic acid sequences, for example, its determination can be made using the algorithm BLAST or the algorithm FASTA developed by Pearson. Based on the algorithm BLAST, programs called BLASTN or BLASTX have been developed (see: http://www.ncbi.nlm.nih.gov).

As used herein, the term "about" is intended to encompass a numerical value that is identical to or numerical values that lie within the range of a certain variation of the following number, for example, within the range of a variation of ±10%, ±9%, ±8%, ±7%, ±6%, ±5%, ±4%, ±3%, ±2%, ±1%, ±0.5%, ±0.4%, ±0.3%, ±0.2%, ±0.1%, ±0.05%, or ±0.01% of the given value, but with no limitations thereto.

*Acremonium chrysogenum* has a cephalosporin biosynthesis pathway that yields cephalosporin C (CPC) as a final product. CPC is synthesized from the precursors L-2-aminoadipate, L-cysteine, and L-valine. It starts with the condensation of the three amino acids to form N-[(5S)-5-amino-5-carboxylpentanoyl]-L-cysteinyl-D-valine. The ring closure of the linear tripeptide leads to the formation of bicyclic isopenicillin N which is then epimerized to penicillin N. This antibiotic is converted to the cephalosporin precursor, DAOC, by conversion of a penam ring to a cephem ring with the catalysis of expandase or DAOC synthase encoded by CefEF gene.

Deacetoxycephalosporin C (DAOC) is represented by the following Chemical Formula 1 and is a precursor for the cephalosporin intermediate 7-amino deacetoxy cephalosporanic acid (7-ADCA; Chemical Formula 2):

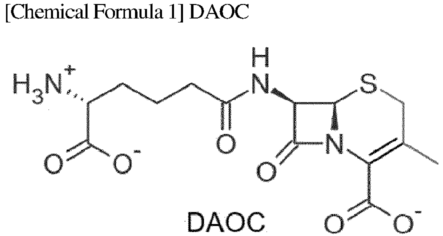

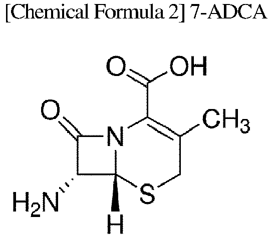

The term "DAOC", as used herein, is construed to encompass DAOC (deacetoxycephalosporin C (DAOC) and/or a pharmaceutically acceptable salt thereof. Also, the term "ADCA" is construed to encompass 7-amino deacetoxy cephalosporanic acid (ADCA) itself and/or a pharmaceutically acceptable salt thereof.

As used herein, the wording "improved DAOC productivity" means increased DAOC production, decreased yield of the byproduct deacetylcephalosporin C (DAC) during DAOC production, and/or a decrease in DAC/DAOC ratio (%).

As used herein, the wording "a culture of a microorganism" may be obtained by culturing the microorganism in a medium. The culture may be in any state; for example, the culture may be a liquid culture and/or a solid culture, but not limited thereto.

As used herein, the wording "a cultured medium" may be obtained from a culture of a microorganism or obtained after culturing a microorganism in a medium. The cultured medium may comprise substances produced and/or secreted by a microorganism cultured. In a specific embodiment, the cultured medium may comprise a cell-free culture where a microorganism is removed from its culture, but not limited thereto.

Below, a detailed description will be given of the present disclosure.

An aspect provides a novel deacetoxycephalosporin C (DAOC)-producing microorganism.

In an embodiment, the DAOC-producing microorganism may be a mutant *Acremonium chrysogenum* microorganism including *Streptomyces clavuligerus-derivative* transferase or a gene coding therefor. The DAOC-producing microorganism may have improved DAOC productivity, compared to non-modified microorganisms. As used herein, the wording "improved or excellent DAOC productivity" refers to high DAOC production and/or high DAOC purity (low yield of byproducts). Hence, the DAOC-producing microorganism is characterized by the high purity of DAOC, with greater production of DAOC and/or lower yield of byproducts such as DAC than non-modified microorganisms.

In an embodiment, the *Streptomyces clavuligerus-derived* aminotransferase may be aminotransferase class V-fold PLP-dependent enzyme (CefD; GenBank Accession No. WP_003952494.1; 398 aa; SEQ ID NO: 1), but is not limited thereto.

In other embodiments, the *Streptomyces clavuligerus-derived* aminotransferase may include a C-terminal fragment of an enzyme responsible for converting isopenicillin N to penicillin N in the host cell *Acremonium chrysogenum,* for example, a C-terminal fragment of isopenicillin N-CoA-synthetase (CefDl; GenBank Accession No. CAD45625.1; 609 aa). The C-terminal fragment may comprise 3 to 30, 3 to 20, 3 to 15, 3 to 12, 3 to 10, 5 to 30, 5 to 20, 5 to 15, 5 to 12, 5 to 10, 8 to 30, 8 to 20, 8 to 15, 8 to 12, or 8 to 10 consecutive C-terminal amino acid residues of isopenicillin N-CoA synthetase of *Acremonium chrysogenum.* The consecutive amino acid residues may contain the C-terminal tripeptide (PRL) responsible for peroxisome targeting signal 1 (PTS 1). In an embodiment, the C-terminal fragment containing PTS 1 may comprise the amino acid sequence of SEQ ID NO: 2 (SGQSAARPRL), but with no limitations thereto. The PTS 1-containing C-terminal fragment may be linked to the C-terminus of the *Streptomyces clavuligerus-derived* aminotransferase. The polypeptide covering the C-terminal fragment comprising the *Streptomyces clavuligerus-derived* aminotransferase and PTS 1 may be represented by SEQ ID NO: 3, but with no limitations thereto.

In an embodiment, the mutant *Acremonium chrysogenum* microorganism may be a transformant constructed by introducing into the host cell *Acremonium chrysogenum* a polynucleotide comprising a nucleic acid sequence coding for a polypeptide (e.g., the amino acid sequence of SEQ ID NO: 3) comprising the *Streptomyces clavuligerus-derived* aminotransferase and the PTS 1-containing C-terminal fragment, or a recombinant vector carrying the polynucleotide. With respect to the transformant, the polynucleotide introduced thereinto may exist in any form without particular limitations thereto. For example, the polynucleotide may be integrated into the genome or chromosome of the host cell or may replicate and/or be expressed by itself extrachromosomally. The nucleic acid sequence may comprise codons optimized for *Acremonium chrysogenum.* In an embodiment, the nucleic acid sequence may be represented by SEQ ID NO: 4, but is not limited thereto. The recombinant vector may be an expression vector that can be expressed in *Acremonium chrysogenum.*

As a host cell, *Acremonium chrysogenum* is a microorganism with ability to produce deacetoxycephalosporin C (DAOC), which is one of raw materials of cephalosporins, and may be a wild-type or mutant microorganism. The mutant microorganism as a host cell may be an *Acremonium chrysogenum* microorganism to which a mutation designed to increase DAOC productivity has been introduced.

In an embodiment the mutation designed to increase DAOC productivity may be at least one selected from the following mutations, but is not limited to:
deletion of the CefEF and/or CefG gene of the host cell; and
introduction of a foreign CefE gene.

As used herein, the term "foreign CefE gene" refers to a CefE gene derived from at least one of various microorganisms other than *Acremonium chrysogenum,* for example, at least one (e.g., 1, 2, 3, 4, 5, 6, 7, or 8) selected from the group consisting of *Amycolatopsis lactamdurans, Gordonia rubripertincta, Mycobacterium abscessus, Microbacterium hydrocarbonoxydans, Nannocystis exedens, Pseudomonas synringae, Streptomyces clavuligerus,* and *Sphingomonas dokdonensis,* but with no limitations thereto.

In an embodiment, the foreign CefE gene may comprise the nucleic acid sequence of GenBank Accession No. NID Z13974.1 as derived from *Amycolatopsis lactamdurans,* the nucleic acid sequence of GenBank Accession No. NID CP022580.1 as derived from *Gordonia rubripertincta,* the nucleic acid sequence of GenBank Accession No. NID FVPM01000026.1 as derived from *Mycobacterium abscessus,* the nucleic acid sequence of GenBank Accession No. NID JYJB01000010.1 as derived from *Microbacterium hydrocarbonoxydans,* the nucleic acid sequence of GenBank Accession No. NID FOMX01000018.1 as derived from *Nannocystis exedens,* the nucleic acid sequence of GenBank Accession No. NID AOJT01001469.1 as derived from *Pseudomonas synringae,* the nucleic acid sequence of Genbank Accession No. NID DS570624.1 as derived from *Streptomyces clavuligerus,* the nucleic acid sequence of GenBank Accession No. NID NBBI01000005.1 as derived from *Sphingomonas dokdonensis,* but is not limited thereto.

In an embodiment, the foreign CefE gene may be the CefE gene (SEQ ID NO: 5) of *Mycobacterium abscessus,* the CefE gene (SEQ ID NO: 6) of *Sphingomonas dokdonensis,* or a combination thereof, but is not limited thereto.

In some embodiments, in addition to the mutation attributed to the introduction of a polynucleotide comprising a nucleic acid sequence coding for the amino acid sequence of SEQ ID NO: 3 and a recombinant vector carrying the polynucleotide as described in the foregoing, the mutant *Acremonium chrysogenum* microorganism provided herein may further comprise at least one mutation selected from among:
deletion of the CefEF and/or CefG gene thereof; and
introduction of a foreign CefE gene.

The foreign CefE gene is as defined above.

As used herein, the wording "deletion of a gene" means the removal of part or entirety (i.e., from start codon to stop codon) of the nucleotide sequence from each of CefEF and/or CefG identified in the chromosomal sequence of a microorganism, but is not limited thereto.

In an embodiment, the mutant *Acremonium chrysogenum* microorganism may be a strain deposited under accession No. KCTC 14989BP, but is not limited thereto.

The mutant *Acremonium chrysogenum* microorganism provided herein may have DAOC productivity. The mutant *Acremonium chrysogenum* microorganism may have improved DAOC productivity (increased DAOC production and/or increased DAOC purity (e.g., decreased DAC production), that is, decreased DAC/DAOC ratio (%)) and/or increased 7-ADCA productivity, compared to non-modified microorganisms. The term "non-modified microorganism" refers to a wild-type *Acremonium chrysogenum* microorganism or an *Acremonium chrysogenum* microorganism that has not undergone the aforementioned mutation, i.e., the mutation attributed to the introduction of a polynucleotide comprising a nucleotide sequence coding for the amino acid sequence of SEQ ID NO: 3 or a recombinant vector carrying the polynucleotide.

Another aspect provides a composition, comprising the mutant *Acremonium chrysogenum* microorganism and/or a culture of the microorganism or a cultured medium of the culture, for producing DAOC and/or 7-ADCA.

A further aspect provides a method for constructing an *Acremonium chrysogenum* with improved DAOC productivity and/or for improving DAOC productivity in an *Acremonium chrysogenum* microorganism, the method comprising a step of introducing into an *Acremonium chrysogenum* microorganism a polynucleotide comprising a nucleic acid sequence coding for a polypeptide (e.g., the amino acid sequence of SEQ ID NO: 3) comprising *Streptomyces clavuligerus-derived* aminotransferase and a PTS1-containing C-terminal fragment. The *Acremonium chrysogenum* thus constructed exhibits improved DAOC productivity, compared to an *Acremonium chrysogenum* microorganism which has not undergone the introduction thereto of a polynucleotide comprising a nucleic acid sequence coding for a polypeptide (e.g., the amino acid sequence of SEQ ID NO: 3) comprising *Streptomyces clavuligerus-derived* aminotransferase and a PTS1-containing C-terminal fragment.

The method may further comprise, before, after, or concurrently with the step of introducing the polynucleotide, at least one selected from the following steps:
deletion of a CefEF and/or CefG gene of the host cell; and
introduction of a foreign CefE gene.

The foreign CefE gene is as defined above.

A yet further aspect provides a method for producing deacetoxycephalosporin C (DAOC), the method comprising a step of culturing the mutant *Acremonium chrysogenum* microorganism. The culturing step may be carried out in a medium and/or condition that allows for the production of DAOC. The method may further comprise a step of recovering (or separating) DAOC from the culture or a cultured medium of the culture.

The mutant *Acremonium chrysogenum* microorganism provided herein has improved DAOC productivity. In an embodiment, the mutant *Acremonium chrysogenum* microorganism may increase in DAOC productivity by 3% or more, 5% or more, 7% or more, 10% or more, 12% or more, or 15% or more, compared to non-modified microorganisms.

In other embodiments, the mutant *Acremonium chrysogenum* microorganism may have a DAC/DAOC (%) of 10 % or less, 9.5 % or less, 9 % or less, 8.5 % or less, 8 % or less, 7.5 % or less, 7 % or less, 6.5 % or less, 6 % or less, 5.5 % or less, 5 % or less, 4.5 % or less, 4 % or less, 3.9 % or less, 3.8 % or less, 3.7 % or less, 3.6 % or less, or 3.5 % or less, and/or may have a DAC/DAOC (%) of 90 % or less, 85 % or less, 80 % or less, 75 % or less, 70 % or less, 65 % or less, 60 % or less, 55 % or less, 50 % or less, 45 % or less, 40 % or less, 39 % or less, 38 % or less, 37 % or less, 36 % or less, or 35 % or less, based on the DAC/DAOC (%) (100%) of the non-modified microorganism, but with no limitations thereto.

Another aspect provides a composition for producing 7-aminodeacetoxycephalosporanic acid (7-ADCA), the composition comprising at least one selected from the group consisting of the mutant *Acremonium chrysogenum* microorganism, a culture of the mutant microorganism, a cultured medium thereof of the culture, and DAOC recovered (separated, purified) from the culture and/or cultured medium.

Another aspect provides a composition for use in producing 7-aminodeacetoxycephalosporanic acid (7-ADCA), wherein the composition comprises at least one selected from the group consisting of the mutant *Acremonium chrysogenum* microorganism, a culture of the mutant microorganism, a cultured medium thereof of the culture, and DAOC recovered (separated, purified) from the culture and/or cultured medium.

Yet another aspect provides a method for producing 7-aminodeacetoxycephalosporanic acid (7-ADCA), the method comprising a step of treating a culture of the mutant *Acremonium chrysogenum* microorganism, a cultured medium of the culture, or DAOC recovered (separated, purified) therefrom with cephalosporin C (CPC) acylase.

The method may further comprise a step of culturing the mutant *Acremonium chrysogenum* microorganism, before the step of treatment with CPC acylase. The culturing step is as described in the foregoing and the method may further comprise a step of recovering DAOC from a culture of the microorganism or a cultured medium of the culture.

The step of treating the mutant microorganism culture containing DAOC with CPC acylase is to prepare 7-ADCA through the conversion catalyzed by CPC acylase. The conversion with CPC acylase may be usually carried out in an aerobic condition such as shaking culture or rotation by a rotating machine. In an embodiment, the conversion reaction is conducted at 5 to 30°C and/or for 1 to 300 minutes, for example, 1 to 120 minutes, but with no limitations thereto. The pH of the culture may be maintained in the range of 3.0 to 9.0 and can be appropriately adjusted with an inorganic or organic acid, an alkaline solution, urea, calcium carbonate, ammonia, etc.

The method for producing 7-ADCA may further comprise a step of recovering (separating, harvesting, purifying, or collecting) 7-ADCA from the reaction mixture after the step of treatment with CPC acylase. In detail, the recovery of 7-ADCA may be performed by a method such as centrifugation, filtration, extraction, spray, evaporation, precipitation, crystallization, electrophoresis, fractional dissolution (e.g., ammonium sulfate precipitation), chromatography (e.g., ion exchange, affinity, hydrophobic, and size exclusion), etc., but with no limitations thereto.

Still another aspect provides a polypeptide (e.g., the amino acid sequence of SEQ ID NO: 3) comprising *Streptomyces clavuligerus-derived* aminotransferase (e.g., SEQ ID NO: 1) and a PTS1-containing C-terminal fragment.

A still further aspect provides a polynucleotide encoding the polypeptide. The polynucleotide may comprise the nucleotide sequence of SEQ ID NO: 4.

Yet still another aspect provides an expression vector carrying the polynucleotide.

A yet still further aspect provides a use for producing a DAOC-producing microorganism and/or improving DAOC productivity of at least one selected from the group consisting of:
a polypeptide (e.g., the amino acid sequence of SEQ ID NO: 3) comprising *Streptomyces clavuligerus-*derived aminotransferase (e.g., SEQ ID NO: 1) and a PTS 1-containing C-terminal fragment;
a polynucleotide (e.g., SEQ ID NO: 4) encoding the polypeptide; and
a recombinant vector (expression vector) carrying the polynucleotide.

In greater detail, provided for producing a DAOC-producing microorganism and/or improving DAOC productivity is a composition comprising at least one selected from the group consisting of:
a polypeptide (e.g., the amino acid sequence of SEQ ID NO: 3) comprising *Streptomyces clavuligerus-*derived aminotransferase (e.g., SEQ ID NO: 1) and a PTS 1-containing C-terminal fragment;
a polynucleotide (e.g., SEQ ID NO: 4) encoding the polypeptide; and
a recombinant vector (expression vector) carrying the polynucleotide.

The DAOC-producing microorganism may be an *Acremonium chrysogenum* microorganism and the improvement of DAOC productivity may be an improvement of DAOC productivity in *Acremonium chrysogenum.*

In the coding regions of the nucleic acid sequences disclosed herein, various alterations or modifications may be made due to their codon degeneracy in consideration of the codons preferred by an organism, for example, *Acremonium chrysogenum,* in which a desired polypeptide is to be expressed, for example, provided that they do not change the amino acid sequence and/or function of the polypeptide expressed from the coding region.

The introduction of the polynucleotide or vector may be carried out using a well-known transformation method appropriately selected by a person skilled in the art. As used herein, the term "transformation" means the introduction of a vector carrying a nucleic acid coding for a target protein (foreign protein) into a host cell in such a way that the protein encoded by the nucleic acid molecule is expressed in the host cell. So long as the transformed nucleic acid molecule is expressed in the host cell, it may be either integrated into the chromosome of the host cell and/or may exist extrachromosomally. Further, the nucleic acid molecule includes DNA and/or RNA encoding the target protein. The nucleic acid molecule may be introduced in any form as long as it can be introduced into the host cell and expressed therein. For example, the nucleic acid molecule may be introduced into the host cell via the form of an expression cassette which is a gene construct including all elements required for its autonomous expression. Typically, the expression cassette includes an expression regulatory element operatively linked to the nucleic acid molecule, such as a promoter, a transcriptional termination signal, a ribosome binding site, and/or a translation termination signal. The expression cassette may be in the form of a self-replicable expression vector. Also, the nucleic acid molecule as it is may be introduced into the host cell and operably linked to sequences required for expression in the host cell. As used herein, the term "operably linked" means a functional linkage between a nucleic acid molecule and an expression regulatory element (e.g., promoter) so as for the expression regulatory element to control (e.g., initiate) the transcription of the nucleic acid molecule encoding a target protein (foreign protein). The operative linkage may be carried out using a genetic recombination technique known in the art. By way of example, the linkage may be implemented by typical site-specific DNA cleavage and ligation, but is not limited thereto.

So long as it ensures the introduction of the polynucleotide into a host cell (microorganism), any transformation (introduction) may be used in the present disclosure. According to host cells, appropriate selection may be made of transformation techniques known in the art. Examples of the transformation methods known in the art include electroporation, calcium phosphate (CaPO₄) precipitation, calcium chloride (CaCh) precipitation, microinjection, polyethylene glycol (PEG)-mediated uptake, DEAE-dextran-mediated gene transfer, cationic liposome-mediated gene transfer, lipofection, lithium acetate-DMSO method, heat shock, particle gun bombardment, silicon carbide whiskers, and sonication, but are not limited thereto.

As used herein, the term "vector" refers to a DNA construct comprising a nucleotide sequence of a nucleic acid molecule coding for a target protein, which is operatively linked to an appropriate expression regulatory sequence to express the target protein in a suitable host cell. The regulatory sequence may comprise a promoter that can initiate transcription, an optional operator sequence for regulating the transcription, a sequence encoding a suitable mRNA ribosome binding site, and/or a sequence regulating the termination of transcription and/or translation. After being transformed into the suitable host cell, the vector may replicate or function independently of the host genome or may be integrated into the genome of the host cell.

The vector available in the present invention is not particularly limited as long as it is able to replicate in the host cell, and any vector known in the art may be used. Examples of conventional vectors may include a natural or recombinant plasmid, cosmid, virus, and bacteriophage. For instance, pWE15, M13, MBL3, MBL4, IXII, ASHII, APII, t10, t11, Charon4A, or Charon21A may be used as a phage vector or cosmid vector, and examples of plasmid vectors include pBC types (e.g., pBC-KS(+)), pBR types (e.g., pBR322, pBR325), pUC types (e.g., pUC 1 18 and pUC119), pBluescriptII types, pGEM types, pTZ types, pCL types, pET types (e.g., pET-22b(+)), Bacillus subtilis-derived plasmids (e.g., pUBl 10, pTPS), but with no limitations thereto.

The vector may further include a selection marker for indicating insertion into the host cells. The selection marker is adapted to select transformed cells, that is, to determine whether the polynucleotide is inserted or not, and may be selected from genes leading to selectable phenotypes such as drug resistance, auxotrophy, resistance to cytotoxic agents, or expression of surface proteins. In the presence of a selective agent, only the cells expressing the selection marker are alive or exhibit a different expression trait and thus can be selected.

Herein, the CefDl nucleic acid sequence of the codon-optimized CefD1 sequence was first hybridized with a PTS1 nucleic acid sequence, and the resulting CefD-PTS1 fusion gene is introduced into *Acremonium chrysogenum,* whereby the existing steps accounting for CefDl, CefD2, and thioesterase functions are reduced into a single step to establish an optimized metabolic pathway through which the target product DAOC can be increased to a significant level, with the concomitant remarkable reduction of the content of the impurity DAC during DAOC production.

### BRIEF DESCRIPTION OF THE DRAWINGS

FIG. 1 is a cleavage map of pB-HFD7px.
FIG. 2 is a graph showing DAOC production (g/L) and ratios of the impurity DAC (DAC/DAOC, %) during the DAOC production process in mutant microorganisms according to an embodiment.
FIG. 3 is a plot of DAOC production (g/L) against culture periods of time in a mutant microorganism according to an embodiment.
FIG. 4 is a plot of ratios of the impurity DAC generated during the DAOC production process to DAOC (DAC/DAOC, %) against culture periods of time in a mutant microorganism according to an embodiment.

### Examples

A better understanding of the present disclosure may be obtained in light of the following examples which are set forth to illustrate, but are not to be construed to limit, the present disclosure. It is apparent to those skilled in the art that the Examples described below may be modified without departing from the essential gist of the disclosure.

### EXAMPLE 1: Construction of D7px-Introduced Microorganism

### 1.1. Preparation of parent strain

For use as a parent strain to which D7px was to be introduced, a deacetoxycephalosporin C (DAOC)-producing *Acremonium chrysogenum* strain was prepared with reference to the Examples in Korean Patent No. 10-2194740 (incorporated herein by reference).

In brief, first, strains were prepared by introducing into a CefEF/CefG-deficient *Acremonium chrysogenum* strain with high CPC production performance (accession no. KCTC 13922BP) a DNA (SEQ ID NO: 5) coding for CefE3 (GenBank PID SKX81615.1) of *Mycobacterium abscessus* (3-7 strain) and a DNA SEQ ID NO: 6) coding for CefE8 (GenBank PID OWK28829.1) of *Sphingomonas dokdonensis* (8-60 strain). According to the "transformation assay" disclosed in Korean Patent No. 10-2194740, protoplasts of the prepared 3-7 and 8-60 strains were constructed and counted. For each strain, 1×10⁷ protoplasts were mixed with 250 µL of a 60% (w/v) PEG solution and left for 40 minutes in ice. Then, the protoplasts were further added and mixed with 2.5 mL of a 60% PEG solution, left for 40 minutes at room temperature, and spread over LB-source plates supplemented with an antibiotic. The plates were incubated at 28°C for 14 days until colonies were formed.

The cells thus formed were examined for the successful introduction of both of CefE3 from *Mycobacterium abscessus* and CefE8 from *Sphingomonas dokdonensis* thereto. In this regard, the CefE3 genes were detected using a primer set of SEQ ID NO: 7 (5'-cgcttgagca gacatcacca tgacggacat cggtgaac-3') and SEQ ID NO: 8 (5'-gctaagcttt tatcagccga cggttatggc-3') for *Mycobacterium abscessus,* and the CefE8 genes were detected using a primer set of SEQ ID NO: 9 (5' -cgcttgagca gacatcacca tgcatcgcgc gggcggc-3') and SEQ ID NO: 10 (5'-gctaagcttt tatcacttct tgatgagac-3') for *Sphingomonas dokdonensis.* As a result, the cell was identified as a new fusion strain (named E3-E8) having both of CefE3 gene from *Mycobacterium abscessus* and CefE8 gene from *Sphingomonas dokdonensis* introduced thereinto.

### 1.2. D7px gene synthesis and cloning

A nucleotide sequence (hereinafter referred to as "D7") resynthesized on the basis of the CefD protein sequence of the cephalosporin-producing actinomyces *Streptomyces clavuligerus,* with codon modifications optimized for *Acremonium chrysogenum* and a coding sequence for 10 consecutive amino acid residues (inclusive of the tripeptide responsible for peroxisome targeting signal 1 (PTS1)) at the C-terminal region of the CefD1 protein of *Acremonium chrysogenum* were prepared. The coding sequence for PTS1 sequence-containing 10 amino acid residues (SEQ ID NO: 2) of CefDl was linked to the 3'-terminus of D7 sequence to give a hybrid sequence named D7px (amino acid sequence: SEQ ID NO: 3; and DNA sequence: SEQ ID NO: 4).

To construct D7px, the sequence of CefD of *Streptomyces clavuligerus* was codon optimized for expression in *Acremonium chrysogenum* through base sequencing and the CefDl sequence of the codon-optimized CefD1 sequence was first hybridized with a PTS1 sequence. The *Acremonium chrysogenum* strain having D7px introduced thereinto exhibited an optimized metabolic pathway wherein the existing steps accounting for CefDl, CefD2, and thioesterase functions were reduced into a single step. Through the optimized pathway, the target product DAOC was increased to a significant level, with the concomitant remarkable reduction of the content of the impurity DAC during DAOC production.

The nucleotide sequence of D7px and the protein sequence encoded thereby are as follows:
Amino acid sequence of D7 (398 aa) (SEQ ID NO: 1) 
MAVADWEEAR GRMLLDPTVV NLNTGSGGPL PRSAFERVTG FRAHLAAEPM
DFLLREVPAL LWQARESLAR LIGGDPLRLA LATNVTAAVN LVASSLRLEA PGEILLSDDE
YTPMRWCWER VARRHGLELR TFRLPELPSD PAEITAAAVA AMGPRTRLFF FSHVVSTTGL
ILPAAELCEE ARARGITTVV DGAHAPGFLD LDLSRIPCDF YAGSGHKWLL APTGVGFLHL
APGRLEELEP TQVSWAYEPP EGSGPPAARD RFGSTPGLRR LECEGTRDIC PWLATPESID
FQAELGPGAI RARRRELTDH ARRLLADRPG RTLLTPDSPE LSGGMVAYRL PPGTDAAELR
RGLWERFRIE AAVAEQPPGP VLRISANFYT TEEEIDRLAD ALDALTGE
Amino acid sequence of 10-mer CefDl region comprising PTS1 sequence (10 aa) (SEQ ID NO: 2)
   SGQSAARPRL
Amino acid sequence of D7px (408 aa) (SEQ ID NO: 3) 
MAVADWEEAR GRMLLDPTVV NLNTGSGGPL PRSAFERVTG FRAHLAAEPM
DFLLREVPAL LWQARESLAR LIGGDPLRLA LATNVTAAVN LVASSLRLEA PGEILLSDDE
YTPMRWCWER VARRHGLELR TFRLPELPSD PAEITAAAVA AMGPRTRLFF FSHVVSTTGL
ILPAAELCEE ARARGITTVV DGAHAPGFLD LDLSRIPCDF YAGSGHKWLL APTGVGFLHL
APGRLEELEP TQVSWAYEPP EGSGPPAARD RFGSTPGLRR LECEGTRDIC PWLATPESID
FQAELGPGAI RARRRELTDH ARRLLADRPG RTLLTPDSPE LSGGMVAYRL PPGTDAAELR
RGLWERFRIE AAVAEQPPGP VLRISANFYT TEEEIDRLAD ALDALTGESG QSAARPRL
D7px-encoding DNA sequence (SEQ ID NO: 4)

atggccgttg cggactggga ggaggcgcgc ggccgcatgc tcctggatcc caccgttgtc 60

aatctaaaca cggggtcggg aggcccgctc cccaggtccg ccttcgagag ggtcacaggt 120

ttccgtgccc acctggccgc ggagccgatg gacttcctgc ttcgcgaggt gcctgctctt 180

ctctggcagg ccagggagag cctcgcccgc ctgatcggtg gcgatccctt gcggctcgcc 240

ctggcgacca atgtcacggc agccgtgaat ctggtcgcca gttcgctgcg tctcgaggca 300

ccgggtgaga ttcttctgtc ggacgacgaa tacacaccca tgagatggtg ctgggagcgc 360

gtggcccgta ggcatggctt ggagctccgc acattccgcc tgccagagct gccaagcgat 420

ccggctgaaa tcactgcagc cgcagttgct gcaatgggac cgcggacgcg actctttttc 480

ttctcccacg tcgtttcgac cacgggtctc atactccccg ctgcggagct ctgcgaagag 540

gcccgagccc gtggcatcac gacggtcgtc gatggcgccc atgcccctgg ttttctggat 600

ctcgacctca gccgtatccc gtgcgacttt tacgccggtt ccggtcataa gtggctcctg 660

gcacccactg gcgttggctt cctccacttg gcgccgggcc gcctagagga actcgagcct 720

acacaggtct catgggccta cgagcctcca gagggctcgg gccctccggc ggcgcgagac 780

cggtttggga gtaccccggg cctgcggagg ctcgagtgcg agggcacgcg agatatctgc 840

ccctggctcg ccacccccga aagtatcgac ttccaagcag agctaggtcc cggggctatc 900

cgtgcgcgtc gtcgcgagct gaccgaccat gcgcggcgcc tgctcgccga ccgtcctggg 960

aggaccctcc ttactcctga ctcgccggag ctctcaggcg gaatggttgc ttacaggttg 1020

ccacccggca ccgacgcagc cgagctgcgc cgtggcctgt gggagcgatt tcgcatcgag 1080

gccgccgtcg ccgaacagcc gccgggcccc gtgctacgca tctccgcgaa tttctatacc 1140

accgaggagg agattgatcg actcgccgac gccctcgacg cccttacggg agagtccggc 1200

cagtcggcgg ctcggccaag actctaatga 1230

For cloning the D7px gene, the gene amplification was conducted using PCR according to the manual packed along with the commercial DNA polymerase product. Usually, Pfu-X polymerase (Solgent, Korea) was used as a DNA polymerase. Restriction enzymes, T4 DNA ligase, and the Klenow fragment were purchased from NEB (USA) and used according to the accompanying manuals. Use was made of QIAprep Spin Miniprep Kit for plasmid DNA purification, QIAquick PCR Purification Kit for PCR product purification, and QIAquick Gel Extraction Kit (Qiagen, Netherland) for DNA extraction from agarose gel.

For use in cloning, E. coli DH5alpha cells (Thermo Fisher) were made competent. In this regard, heat shock was imparted to the cells. Then, the E. coli cells were inoculated into LB broth (BD Difco) and cultured until OD₆₀₀ reached 0.4-0.6, followed by centrifugation (4°C, 4000 rpm) to recover the cell mass. The recovered cell mass washed four times with iced 0.1 M calcium chloride to prepare competent cells. A plasmid DNA to be used for heat-shock transformation (generally called subcloning plasmid for transformation) was prepared in an amount of 100 to 500 ng. Together with the plasmid DNA, 100 µL of the competent cells was incubated for 30 minutes in ice. Immediately after heat shock at 42°C for 30 sec, the mixture was cooled in ice for 2 minutes and then added with 1 mL of LB broth. The cells were cultured at 37°C for 1 hour, spread over an LB plate containing an antibiotic, and incubated overnight at 37°C in a stagnant mode to obtain a transformant.

The gene of D7px was amplified from a template obtained by synthesis request. Amplification of D7px was conducted using a primer set of 5'-CGCTTGAGCAGACATCACCATGGCCGTTGCGGACTGGG-3' (SEQ ID NO: 11) and 5'-TATGAATTCTCATTAGAGTCTTGGCCGAG-3' (SEQ ID NO: 12). The promoter PEP3 (SEQ ID NO: 13) was amplified from pB-HCXEP3 (SEQ ID NO: 16) using a primer set of 5'-GCAACTAGTGCGGCCGCCCTTGTATCTCTACACACAGGC-3' (SEQ ID NO: 14) and 5'-GGTGATGTCTGCTCAAGCG-3' (SEQ ID NO: 15).

The amplified D7px gene and promoter PEP3 were linked to each other by sewing PCR, taking advantage of the common sequence therebetween. To this end, PCR was performed using a primer set of SEQ ID NOS: 12 and 14, with the amplified D7px fragment and PEP3 fragment serving as a template. The resulting amplicon was a PEP3-D7px fragment in which PEP3 was linked to D7px. To establish a terminator sequence, the PEP3-D7px fragment was digested with SpeI and HindIII and inserted into at the same restriction enzyme site of the terminator vector pB-TtrpC (SEQ ID NO: 17) to give pB-D7pxcast (SEQ ID NO: 18).

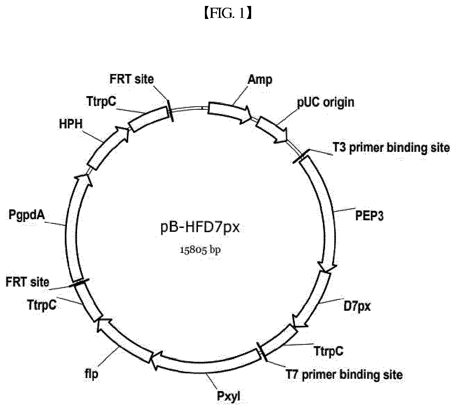

From pB-D7pxcast, a D7 gene cassette was amplified using a primer set of T3 (SEQ ID NO: 19) and T7(SEQ ID NO: 20), followed by blunt-end ligation of the amplicon to the PmeI restriction site of pB-HF (SEQ ID NO: 21). The plasmid thus constructed was named pB-HFD7px (SEQ ID NO: 22). This expression vector has the structure depicted in FIG. 1. The plasmid comprises a hygromycin antibiotic marker and is designed to remove the marker with an flp-FRT system.

### 13. Transformation for introduction of D7px gene

The antibiotic marker was removed from the parent strain E3-E8 prepared in Example 1.1. In this regard, the strain was passaged on an LB plate containing an antibiotic and 2% xylose. Colonies appeared after 10 days of incubation and total DNA was extracted from the colonies. The deletion of the marker gene cassette was examined by PCR. These steps were repeated to select variants that were completely deficient in the antibiotic marker gene cassette. Thereafter, the strain was transformed with the pB-HFD7px vector prepared in Example 1.2. The transformants thus obtained were cultured in vitro and compared for DAOC productivity, followed by selection of highly productive strains for use in an expression assay.

The preparation of pB-HFD7px vector for transformation into *Acremonium chrysogenum* was carried out according to the manual of the QIAprep Spin Miniprep Kit (Qiagen, Netherlands).

PEG (polyethylene glycol)-mediated transformation into *Acremonium chrysogenum* was performed. To acquire cell mass, the fungal strain (E3-E8) was spread on an LB plate and incubated at 28°C for 7 days. The mycelia thus obtained were cut with a flame-sterilized scalpel into squares with a side size of 5-7 mm and 4-6 mycelial squares were inoculated into TB broth (12 g/L Tryptone, 24 g/L yeast extract, 9.4 g/L K₂HPO₄, 2.2 g/L KH₂PO₄, 4 g/L glycerol) which was then incubated at 28°C for 3 days. Cell mass was recovered by centrifuging the culture (4°C, 4000 rpm), discarding the supernatant, and washing the cell pellet once with 0.6 M MgSO₄.

For protoplast formation, the cell mass was weighed and the total volume comprising 2% lysing enzyme (Sigma-Aldrich L1412, USA) was adjusted to amount to four times the weight of the cell mass. After reaction at 30°C for 3 hours while shaking at 100 rpm, an overlay was made with the same volume of a separation buffer A (0.6 M sorbitol, 100 mM Tris-Cl, pH 7.0). Centrifugation (4°C, 1,800 g) separated a protoplast layer. The protoplast layer was transferred to a new tube and mixed with the same volume of separation buffer B (1.2 M sorbitol, 100 mM Tris-Cl, pH 7.5), followed by centrifugation (4°C, 1,800 g) to harvest the protoplasts. The supernatant was discarded and the protoplasts were washed once with an MSC buffer (1 M sorbitol, 10 mM MOPS, pH 6.5, 10 mM CaCl₂) before counting under a microscope. As a result, 1×10⁷ protoplasts were acquired.

For transformation, 1×10⁷ protoplasts 1-5 µg of DNA (pB-HFD7px), 50 µL of 60%(w/v) PEG solution (polyethyleneglycol 6000 dissolved at a concentration of 60%(w/v) in MSC buffer) were mixed and incubated for 20 minutes in ice. An additional 500 µL of 60% PEG solution was added to the mixture which was then incubated at room temperature for 20 minutes and spread over LB-sucrose plates (0.8 M sucrose, 2% (w/v) agar) containing an antibiotic suitable for transformant selection. The plates were incubated at 28°C for 14 days to form transformants into pB-HFD7px had been successfully introduced. Since the vector pB-HFD7px illustratively used in this Example was designed so that it cannot exist in the form of a plasmid when introduced into cells, most of the obtained transformants had the D7px gene integrated into the chromosome thereof. However, this is only an example of carrying out the present invention, and the form of a plasmid is not excluded. The introduced gene may exist in any form.

To secure spores, the obtained transformants were cut out with a flame-sterilized scalpel, picked up with forceps, placed in a 1.5 mL e-tube, and added with 200 µL of 0.85% NaCl, followed by disrupting the cells with a pestle. The cells were spread over spore media (starch 24 g/L, glycine 1.2 g/L, polypeptone 4 g/L, yeast extract 0.3 g/L, casein 8 g/L, ammonium sulfate 6 g/L, potassium phosphate dibasic 1.2 g/L, magnesium sulfate 0.6 g/L, agar 20 g/L, pH 7.0) and incubated at 28°C for 14 days. The spores were scraped with a platinum loop, suspended in 20 %(w/v) glycerol, and stored in a -80°C deep freezer.

As such, the mutant *Acremonium chrysogenum* strain having D7px gene introduced thereinto was deposited under the accession number KCTC 14989BP at the Biological Resources Center of the Korea Research Institute of Bioscience and Biotechnology located in Jeongeup-si, Jeollabuk-do, Korea on June 2, 2022.

### EXAMPLE 2. Selection of Strain with High DAOC Productivity

The transformants prepared in Example 1.3 were measured for productions of deacetoxycephalosporin C (DAOC) and deacetylcephalosporin C (DAC) and yield of conversion into 7-ADCA.

For selection of a strain with high DAOC productivity and low DAC yield, the obtained transformants were cultured in vitro in a medium containing sugar 15 g/L, soytone 15 g/L, ammonium sulfate 5 g/L, methionine 10 g/L, calcium carbonate 10 g/L, yeast extract 10 g/L, glucose 5 g/L, magnesium surface 2 g/L, and methyl oleate 50 g/L. The spores (1×10⁷ spores) harvested in Example 1.3 were inoculated into the test tube and incubated at 28°C for 10 days while shaking at 200 rpm, and 0.5 mL of the culture was taken. To analyze productions of DAOC and DAC, 25 µL of the supernatant was mixed with 975 µL of tertiary distilled water and the mixture was filtered through a 0.2 µm filter and subjected to HPLC.

HPLC analysis conditions were as follows: HPLC instrument: Shimadzu LC10Avp, Column: ZORBAX Eclipse Plus C 18(Analytical 4.6 mm×250 mm, 5-Micron), Mobile phase: 20 mM ammonium acetate: acetonitrile (95:5), pH: 7.0, flow rate: 0.8 mL/min, Column temperature: 40°C, and Detector: UV at 220 nm. The HPLC analysis conditions were commonly used to analyze DAOC, DAC, and 7-ADCA in Examples 2 and 3.

In addition, the ratio of DAOC and DAC (DAC/DAOC, %) was calculated by dividing the DAC area value by the DAOC area value. A lower DAC/DAOC value accounts for a higher production of the target product DAOC and a lower production of the impurity DAC during the DAOC production processes.

Treatment of the culture with CPC acylase enzymatically converted DAOC into 7-ADCA. As the CPC acylase, the mutant CPC acylase (PM2 mutant; encoded by SEQ ID NO: 23) disclosed in Korean Patent No. 10-2014-0094150 (incorporated herein by reference) was employed. In more detail, for HPLC analysis, the pH of the DAOC-containing cell culture supernatant prepared in a culture tube was adjusted to 8.0 using 14%(v/v) ammonia water, and liquid-phase CPC acylase was added at a final concentration of 30 U/mL, followed by incubation at 15°C for 1 hour while shaking at 200 rpm. Then, 7-ADCA conversion yield (g/L) was analyzed by HPLC. In greater detail, after centrifugation, 25 µL of the supernatant was taken and 40-fold diluted in 975 µL of tertiary distilled water. The diluted fermentation was filtered through a 0.2-µm filter and then subjected to HPLC. HPLC analysis conditions were as defined above.

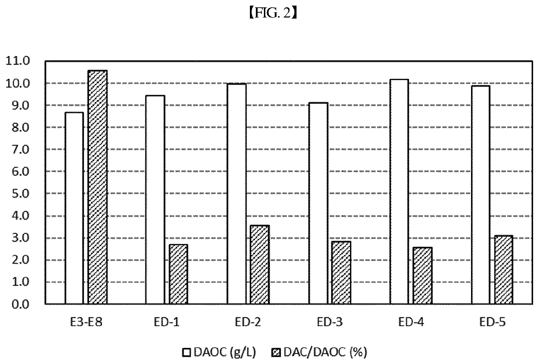

The ratios of the impurity DAC (DAC/DAOC, %) generated during the DAOC production process to DAOC production (g/L) are depicted in FIG. 2 and summarized, together with 7-ADCA conversion yield (g/L), in Table 1.

**TABLE 1**

| Stain | DAOC Production (g/L) | DAC/DAOC (%) | 7-ADCA Conversion (g/L) |
|---|---|---|---|
| E3-E8 (Control) | 8.66 g/L | 10.55 % | 4.59 g/L |
| ED-1 | 9.45 g/L | 2.69 % | 5.02 g/L |
| ED-2 | 9.96 g/L | 3.54 % | 5.26 g/L |
| ED-3 | 9.11 g/L | 2.82 % | 4.86 g/L |
| ED-4 | 10.16 g/L | 2.55 % | 5.38 g/L |
| ED-5 | 9.85 g/L | 3.1 % | 5.25 g/L |

In FIG. 2 and Table 1, E3-E8 was a parent strain (control) without D7px, and ED-1, ED-2, ED-3, ED-4, and ED-5 were all transformed strains obtained by introducing D7px into E3-E8.

As can be seen in FIG. 2 and Table 1, the five transformed test strains all increased by 9-17% in DAOC productivity, compared to the control E3-E8 and exhibited a DAC/DAOC (%) of 2.55-3.54%, which was remarkably decreased, compared to that of the control E3-E8 (about 10.5%). Moreover, as shown in Table 1, the five transformed test strains all increased by about 6-17% in DAOC to 7-ADCA conversion yield, compared to the control E3-E8.

### EXAMPLE 3. DAOC Productivity, DAC/DAOC Ratio, and 7-ADCA Conversion Yield

The ED-4 strain, which was observed to have the highest production of DAOC and the lowest production of the byproduct DAC, was analyzed for DAOC productivity and DAC/DAOC ratio according to culture periods of time. Also, the 7-ADCA conversion yield was measured during the maximum culture period of time. E3-E8 strain was used as a control.

DAOC production was carried out in the sequential steps of primary and secondary seed cultivation and main cultivation. For primary seed cultivation, 4 to 6 colonies according to colony size were inoculated into a primary seed culture broth (bean powder 28.5 g/L, corn steep liquor 25 mL/L, sucrose 35 g/L, glucose 5 g/L, calcium carbonate 5 g/L, endoplasmic reticulum 0.8 mL/L) and incubated at 30°C for 63 hours while shaking at 200 rpm. For secondary cultivation, all of the primary culture was inoculated into a fermentation bath containing a secondary seed culture medium (the primary seed culture medium plus soybean oil 5 mL/L) and cultured for 68 hours initially under the condition of 30°C, 35% DO, 400 rpm, and air 1.0 vvm and during which 6% glucose was fed into the medium and the stirring speed was gradually increased as the cells grew. The main cultivation was carried out by inoculating 200 mL of the secondary seed culture into a fermentation bath containing a main culture medium (peanut powder 23 g/L, corn steep liquor 50 mL/L, methionine 1.5 g/L, dextrin 70 g/L, cornmeal 35 g/L, calcium sulfate 13 g/L, soybean oil 60 mL/L, ammonium sulfate 13 g/L, fructose syrup 9 g/L, calcium carbonate 10 g/L, endoplasmic reticulum 0.5 mL/L) and cultured initially under the condition of 28°C, 35% DO, 400 rpm, and air 1.0 vvm and then under the condition wherein the pH was adjusted into 5.4 to 5.7 using ammonia water and the stirring speed was increased in a stepwise manner as the cells grew. After two days of cultivation, the culturing temperature was lowered from 28°Cto 25°C, and 5% to 10% of soybean oil was supplied in a stepwise manner as the cells grew. The fermentation was completed on day 7 to day 8.

The fermented culture was analyzed for DAOC and DAC production. In this regard, 1 mL of the culture was 100-fold diluted and centrifuged at 14,000 rpm for 10 minutes, and the supernatant was filtered through a 0.2-um syringe filter before use in HPLC analysis. HPLC analysis conditions were as described in Example 2. The ratio of DAOC and DAC (DAC/DAOC, %) was calculated by dividing the DAC area value by the DAOC area value.

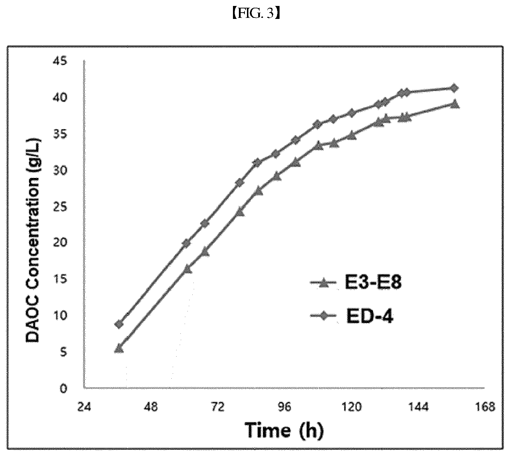

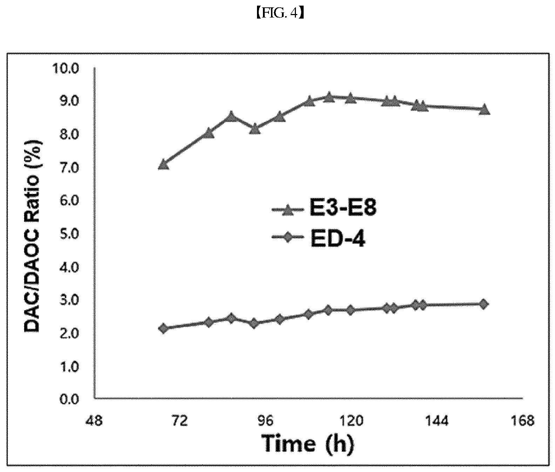

The measurements are given in FIG. 3 and Table 2 for DAOC production (g/L) and FIG. 4 and Table 3 for DAC/DAOC ratio (%):

**TABLE 2**

| **DAOC Concentration (g/L)** | | | |
|---|---|---|---|
| Time(h) | E3-E8 | Time(h) | ED-4 |
| 36.5 | 5.5 | 36.7 | 8.7 |
| 60.8 | 16.4 | 61.0 | 19.9 |
| 67.3 | 18.7 | 67.5 | 22.6 |
| 80.0 | 24.2 | 80.0 | 28.2 |
| 86.3 | 27.1 | 86.5 | 30.9 |
| 92.8 | 29.1 | 93.0 | 32.2 |
| 100.0 | 31.1 | 100.0 | 34.1 |
| 108.0 | 33.4 | 108.3 | 36.2 |
| 113.5 | 33.7 | 113.8 | 37.0 |
| 120 | 34.8 | 120 | 37.8 |
| 130 | 36.6 | 130 | 39.0 |
| 132 | 37.0 | 132.3 | 39.2 |
| 138 | 37.1 | 138.3 | 40.5 |
| 140 | 37.3 | 140 | 40.5 |
| 157 | 39.0 | 157.3 | 41.2 |

**TABLE3**

| **DAC/DAOC Ratio (%)** | | | |
|---|---|---|---|
| Time(h) | E3-E8 | Time(h) | ED-4 |
| 67.3 | 7.1 | 67.5 | 2.1 |
| 80.0 | 8.0 | 80.0 | 2.3 |
| 86.3 | 8.5 | 86.5 | 2.4 |
| 92.8 | 8.2 | 93.0 | 2.3 |
| 100.0 | 8.5 | 100.0 | 2.4 |
| 108.0 | 9.0 | 108.3 | 2.6 |
| 113.5 | 9.1 | 113.8 | 2.7 |
| 120 | 9.1 | 120 | 2.7 |
| 130 | 9.0 | 130 | 2.7 |
| 132 | 9.0 | 132.3 | 2.7 |
| 138 | 8.8 | 138.3 | 2.8 |
| 140 | 8.8 | 140 | 2.8 |
| 157 | 8.7 | 157.3 | 2.9 |

As can be seen in FIGS. 3 and 4 and Tables 2 and 3, the ED-4 strain having D7px gene introduced thereinto was higher in DAOC production and remarkably lower in DAC/DAOC than the control E8-E3 strain.

In addition, enzymatic conversion of DAOC into 7-ADCA was performed by treating the culture with CPC acylase. As the CPC acylase, the mutant CPC acylase (PM2 mutant; encoded by SEQ ID NO: 23) disclosed in Korean Patent No. 10-2014-0094150 (incorporated herein by reference) was employed. In more detail, 14%(v/v) ammonia water was added to a fermentation bath containing the culture after the maximum culturing period of time to adjust the pH to 8.0, and liquid-phase CPC acylase was added at a final concentration of 30 U/mL, followed by incubation at 15°C for 1 hour while shaking at 800 rpm. Then, 7-ADCA conversion yield (g/L) was analyzed by HPLC. In this regard, 1 mL of the fermentation culture was taken and 100-fold diluted before centrifugation at 14,000 rpm for 10 minutes. The supernatant was filtered through a 0.2-µm filter and then subjected to HPLC. HPLC analysis conditions were as defined in Example 2.

Based on the data obtained, final DAOC production, DAC/DAOC ratio, and 7-ADCA conversion yield (g/L) are summarized in in Table 4, below.

**TABLE 4**

| Strain | Time (h) | DAOC Production (g/L) | DAC/DAOC (%) | 7-ADCA Conversion (g/L) |
|---|---|---|---|---|
| E3-E8 (Control) | 157.0 h | 39.0 g/L | 8.70% | 21.1 g/L |
| ED-4 | 157.3 h | 41.2 g/L | 2.90% | 22.4 g/L |

As is understood from data of Table 4, the ED-4 strain having D7px gene introduced thereinto was higher in DAOC production, remarkably lower in DAC/DAOC ratio, and higher in 7-ADCA conversion yield than the control E8-E3 strain.

The mutant *Acremonium chrysogenum* strain having D7px gene introduced thereinto was deposited under the accession number KCTC 14989BP at the Biological Resources Center of the Korea Research Institute of Bioscience and Biotechnology located in Jeongeup-si, Jeollabuk-do, Korea on June 2, 2022.

Based on the above description, it will be understood by those skilled in the art that the present disclosure may be implemented in a different specific form without changing the technical spirit or essential characteristics thereof. Therefore, it should be understood that the above embodiment is not limitative, but illustrative in all aspects. The scope of the present disclosure is defined by the appended claims rather than by the description preceding them, and thus all changes and modifications that fall within metes and bounds of the claims or equivalents of such metes and bounds are therefore intended to be embraced by the claims.

### [Accession Number]

Depositary authority: Biological Resources Center of the Korea Research Institute of Bioscience and Biotechnology
Accession No.: KCTC14989BP
Deposition date: June 02, 2022

## Claims

1. A mutant *Acremonium chrysogenum* microorganism, comprising a polypeptide or a gene coding therefor, the polypeptide comprising:
(1) a CefD protein of *Streptomyces clavuligerus;* and
(2) a C-terminal fragment comprising 8 to 12 consecutive C-terminal amino acid residues of CefDl protein of *Acremonium chrysogenum,*
wherein the C-terminal fragment of CefD 1 protein comprises peroxisome targeting signal 1 (PTS1) and is linked to the C-terminus of the CefD protein.

2. The mutant *Acremonium chrysogenum* microorganism of claim 1, wherein
the CefD protein comprises the amino acid sequence of SEQ ID NO: 1; and/or
the C-terminal fragment of CefDl protein comprises the amino acid sequence of SEQ ID NO: 2.

3. The mutant *Acremonium chrysogenum* microorganism of claim 1 or 2, wherein:
the polypeptide is represented by the amino acid sequence of SEQ ID NO: 3, and /or
the gene coding for the polypeptide comprises the nucleotide sequence of SEQ ID NO: 4.

4. The mutant *Acremonium chrysogenum* microorganism of any one of claims 1 to 3, further comprising at least one mutation selected from among:
deletion of the CefEF and/or CefG gene thereof; and
introduction of a foreign CefE gene; optionally

5. The mutant *Acremonium chrysogenum* microorganism of claim 4, wherein the foreign CefE gene comprises at least one selected from the group consisting of respective CefE genes derived from *Mycobacterium abscessus, Sphingomonas dokdonensis, Amycolatopsis lactamdurans, Gordonia rubripertincta, Microbacterium hydrocarbonoxydans, Nannocystis exedens, Pseudomonas synringae,* and *Streptomyces clavuligerus.*

6. The mutant *Acremonium chrysogenum* microorganism of any one of claims 1 to 5, deposited under accession number KCTC14989BP.

7. The mutant *Acremonium chrysogenum* microorganism of any one of claims 1 to 6, **characterized by** at least one selected from among:
an increase in deacetoxycephalosporin C (DAOC) production, compared to a non-modified microorganism,
a decrease in deacetylcephalosporin C (DAC) production, compared to a non-modified microorganism; and
a decrease in DAC/DAOC ratio (%), compared to a non-modified microorganism.

8. A composition for use in producing deacetoxycephalosporin C (DAOC), the composition comprising the mutant *Acremonium chrysogenum* microorganism of any one of claims 1 to 7.

9. A method for producing deacetoxycephalosporin C (DAOC), the method comprising a step of culturing the mutant *Acremonium chrysogenum* microorganism of any one of claims 1 to 7.

10. A composition for use in producing 7-aminodeacetoxycephalosporanic acid (7-ADCA), the composition comprising at least one selected from the group consisting of the mutant *Acremonium chrysogenum* microorganism of any one of claims 1 to 7, a culture of the mutant *Acremonium chrysogenum* microorganism, a cultured medium of the culture, and DAOC isolated from the culture or the cultured medium.

11. A method for producing 7-aminodeacetoxycephalosporanic acid (7-ADCA), the method comprising a step of treating a culture of the mutant *Acremonium chrysogenum* microorganism of any one of claims 1 to 7, a cultured medium of the culture, or DAOC isolated from the culture or the cultured medium, with cephalosporin C (CPC) acylase.

12. A polypeptide, which is represented by the amino acid sequence of SEQ ID NO: 3.

13. A polynucleotide coding for the polypeptide of claim 10.

14. An expression vector, carrying a polynucleotide coding for a polypeptide comprising the amino acid sequence of SEQ ID NO: 3.

15. A composition for use in constructing a microorganism having improved DAOC productivity, the composition comprising at least one selected from the group consisting of:
a polypeptide represented by the amino acid sequence of SEQ ID NO: 3;
a polynucleotide coding for the polypeptide; and
an expression vector carrying the polynucleotide.
